# EUROPÄISCHE PATENTSCHRIFT

(11) **EP 0 994 849 B1**
(45) Veröffentlichungstag und Bekanntmachung des Hinweises auf die Patenterteilung: **04.09.2002**
(21) Anmeldenummer: 98932174.0
(22) Anmeldetag: 18.06.1998
(51) Int. Cl.: C07C 253/00, C07C 255/50

(54) **VERFAHREN ZUR HERSTELLUNG VON 2,4,5-TRIFLUOR-BENZONITRIL**
METHOD FOR PRODUCTION OF 2,4,5-TRIFLUOROBENZONITRILE
PROCEDE DE PREPARATION DE 2,4,5-TRIFLUOROBENZONITRILE

(30) Priorität: 01.07.1997 DE 19727890
(43) Veröffentlichungstag der Anmeldung: 26.04.2000
(73) Patentinhaber: BAYER AG, 51368 Leverkusen (DE)
(72) Erfinder: HUPPERTS, Achim, D-40217 Düsseldorf (DE); LANTZSCH, Reinhard, D-42115 Wuppertal (DE)
(86) Internationale Anmeldenummer: EP9803732
(87) Internationale Veröffentlichungsnummer: WO99001425

(56) Entgegenhaltungen:
- EP-A- 0 191 185
- EP-A- 0 682 008
- FR-A- 2 632 648
- GB-A- 1 131 501
- "HOUBEN-WEYL Methoden der Organischen Chemie, 4. Auflage, Band X/3, Teil 3" 1965 , GEORG THIEME VERLAG , STUTTGART, DE XP002081804 siehe Seite 30 - Seite 31

## Beschreibung

Die Erfindung betrifft ein Verfahren zum Herstellen von 2,4,5-Trifluor-benzonitril, welches als Ausgangsstoff für Wirkstoffe im Bereich der Medizin und der LandwirtschaftVerwendung findet.

Es ist bekannt, daß man 2,4,5-Trifluor-benzonitril erhält, wenn 1-Brom-2,4,5-trifluor-benzol mit Kupfer(I)-cyanid und N-Methyl-pyrrolidon in einem verschlossenen Reaktionsgefäß mehrere Stunden auf Temperaturen zwischen 170°C und 190°C erhitzt wird (siehe EP-A-191 185). Nach diesem Syntheseverfahren erhält man jedoch ein stark verunreinigtes Produkt, das erst nach Säulenchromatografie in genügender Reinheit isoliert werden kann.

Nach einem etwas abgewandelten Verfahren kann 1-Brom-2,4,5-trifluor-benzol mit Kupfer(I)-cyanid auch bei Verwendung von N,N-Dimethyl-formamid als Lösungsmittel und längerem Erhitzen unter Rückfluß in 2,4,5-Trifluor-benzonitril überführt werden (siehe J. Med. Chem. 31 (1988), 983-991). Die Isolierung von reinem 2,4,5-Trifluor-benzonitril ist in dieser Publikation jedoch nicht beschrieben.

Es ist weiterhin bekannt, daß man 2-Chlor-3,4,5-trifluorbenzonitril erhalten kann, wenn man das entsprechende Amin zunächst mit Natriumnitrit/Salzsäure diazotiert und das erhaltene Produkt anschließend mit äquimolaren Mengen Kupfersulfat und einem Überschuss Kaliumcyanid in wässrigem Ammoniak zum Nitril umsetzt (siehe EP-A-682 008).

Weiter ist die Umsetzung von 2,4-Dichlor-5-fluor-benzonitril mit Kaliumfluorid, gegebenenfalls in Gegenwart von Reaktionshilfsmitteln, wie z.B. Caesiumfluorid und Octadecyl-trimethylammoniumchlorid, in Gegenwart von Verdünnungsmitteln, wie z.B. Dimethylsulfoxid, Tetramethylensulfon (Sulfolan) und Toluol, bei Temperaturen oberhalb von 150°C bekannt (siehe EP-A-431 373, EP-A-433 124, EP-A-497 239, EP-A-635 486). Hierbei wird jedoch meist 2,4,5-Trifluor-benzonitril neben anderen Produkten nur in geringen oder mäßigen Ausbeuten erhalten. Lediglich bei Verwendung von größeren Mengen an Phasentransfer-Katalysatoren, insbesondere von Tetraalkylphosphoniumhalogeniden soll 2,4,5-Trifluor-benzonitril in höheren Ausbeuten erhalten werden (siehe EP-A-557 949).

Ferner ist bekannt, daß 2,4,5-Trifluor-benzonitril in Mischung mit anderen Fluorierungsprodukten auch durch Umsetzung von 2,4-Difluor-benzonitril mit elementarem Fluor bei tiefen Temperaturen erhalten werden kann (siehe EP-A-566 268).

Die Synthese einiger Trihalogenbenzonitrile - nicht jedoch von 2,4,5-Trifluor-benzonitril - ist auch ausgehend von entsprechenden Trihalogenanilinen über die Diazoniumsalze und deren Umsetzung mit Metallcyaniden beschrieben worden (zu diesem "Sandmeyer-Verfahren" siehe GB-A-951 770, Coll. Czech. Chem. Com. 42 (1977), 2001-2017). Qualität bzw. Ausbeute der so erhaltenen Produkte sind jedoch nicht zufriedenstellend.

Aufgabe der vorliegenden Erfindung ist es, ein Verfahren bereitzustellen, wodurch 2,4,5-Trifluor-benzonitril in hoher Reinheit und mit großen Ausbeuten ausgehend von 2,4,5-Trifluor-anilin hergestellt werden kann.

Die Aufgabe wird gelöst durch ein Verfahren zum Herstellen von 2,4,5-Trifluor-benzonitril der Formel (I) 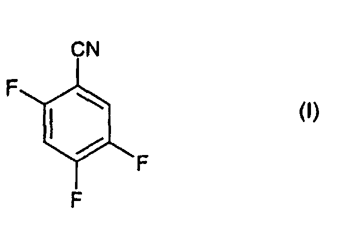 worin 2,4,5-Trifluor-anilin der Formel (II) 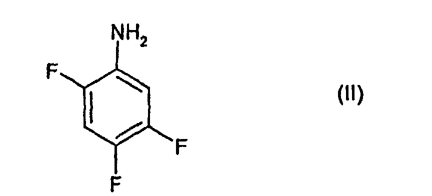 in einem ersten Schritt A) mit einem Nitrosierungsmittel in Gegenwart eines Verdünnungsmittels umgesetzt wird
und worin in einem zweiten Schritt B) das im Schritt A) erhaltene Reaktionsprodukt mit einem Alkalimetallcyanid in Gegenwart einer Übergangsmetallverbindung, wobei die Übergangsmetallverbindung zwischen 0,01 und 0,5 Mol bezogen auf 1 Mol des im ersten Schritt eingesetzten 2,4,5-Trifluor-anilins eingesetzt wird, eines Säureakzeptors und eines Verdünnungsmittels umgesetzt wird.

Überraschenderweise kann nach dem erfindungsgemäßen Verfahren 2,4,5-Trifluor-benzonitril mit großer Ausbeute und in hoher Reinheit erhalten werden.

Die Reaktionstemperaturen können bei der Durchführung des erfindungsgemäßen Verfahrens in einem größeren Bereich variiert werden. Im allgemeinen arbeitet man im ersten Schritt bei Temperaturen zwischen -20°C und +30°C, vorzugsweise zwischen -10°C und +20°C, im zweiten Schritt im allgemeinen bei Temperaturen zwischen -10°C und +40°C, vorzugsweise zwischen 0°C und +30°C.

Die beiden Schritte des erfindungsgemäßen Verfahrens werden im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Die Ausgangsverbindung 2,4,5-Trifluor-anilin ist bekannt und leicht herstellbar (siehe GB-A-11 31 501, EP-A-415 585).

Der erste Schritt des erfindungsgemäßen Verfahrens wird unter Verwendung eines Nitrosierungmittels durchgeführt. Es kommen hierbei die üblichen zur Herstellung von Diazoniumsalzen geeigneten Nitrosierungsmittel in Betracht. Als Beispiele hierfür seien genannt:

Alkalimetallnitrite, wie z.B. Natrium- und Kaliumnitrit (in Gegenwart einer Säure, wie z.B. Schwefelsäure, Methansulfonsäure, Ameisensäure oder Essigsäure), Alkylnitrite, wie z.B. Methyl-, Ethyl-, n- oder i-Propyl-, n-, i-, s- oder t-Butyl-, n-, i-, s-oder t-Pentyl-nitrit, ferner auch Nitrosylschwefelsäure.

Nitrosylschwefelsäure wird als Nitrosierungsmittel bei der Durchführung des ersten Schrittes des erfindungsgemäßen Verfahrens besonders bevorzugt.

Der zweite Schritt des erfindungsgemäßen Verfahrens wird unter Verwendung eines Alkalimetallcyanids durchgeführt. Als Alkalimetallcyanide werden vorzugsweise Natriumcyanid oder Kaliumcyanid eingesetzt. Zusätzlich wird eine Übergangsmetallverbindung, vorzugsweise eine Kupferverbindung, wie z.B. Kupfercyanid oder Kupfersulfat verwendet. Überraschenderweise kann die Übergangsmetallverbindung weit unterhalb der stöchiometrischen Menge, d.h. also in katalytischer Menge eingesetzt werden.

Der zweite Schritt des erfindungsgemäßen Verfahrens wird weiterhin in Gegenwart eines Säureakzeptors durchgeführt. Als Säureakzeptoren kommen im allgemeinen die üblichen anorganischen oder organischen Basen oder Säurebindemittel in Betracht. Hierzu gehören vorzugsweise Alkalimetall- oder Erdalkalimetallacetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calciumamid, Natrium-, Kalium- oder Calciumcarbonat, Natrium-, Kalium- oder Calciumhydrogencarbonat, Lithium-, Natrium-, Kalium- oder Calciumhydrid, Lithium-, Natrium-, Kalium- oder Calcium-hydroxid, Natrium- oder Kaliummethanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyldiisopropylamin, N,N-Dimethylcyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Vorzugsweise werden Alkalimetall- oder Erdalkalimetallcarbonate oder -hydrogencarbonate, wie z.B. Natrium- oder Kalium(hydrogen)carbonat eingesetzt.

Das erfindungsgemäße Verfahren wird in Gegenwart eines Verdünnungsmittel durchgeführt. Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Carbonsäure, wie z.B. Ameisensäure, Essigsäure oder Propionsäure; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Carbonsäuren werden als Verdünnungsmittel bei der Durchführung des ersten Schrittes des erfindungsgemäßen Verfahren besonders bevorzugt. Bei der Durchführung des zweiten Schrittes des erfindungsgemäßen Verfahrens wird vorzugsweise Wasser als weiteres Lösungsmittel verwendet.

Zur Durchführung des ersten Schrittes des erfindungsgemäßen Verfahrens setzt man auf 1 Mol 2,4,5-Trifluor-anilin der Formel (II) im allgemeinen zwischen 0,9 und 1,2 Mol, vorzugsweise zwischen 1,0 und 1,1 Mol eines Nitrosierungsmittels ein.

In einer bevorzugten Ausführungsform des ersten Schrittes des erfindungsgemäßen Verfahrens wird das 2,4,5-Trifluor-anilin der Formel (II) in einem geeigneten Verdünnungsmittel vorgelegt und das Nitrosierungsmittel wird unter Rühren langsam eindosiert. Die Reaktionsmischung wird dann weiter gerührt, bis die Umsetzung gemäß des ersten Schrittes praktisch abgeschlossen ist.

Zur Durchführung des zweiten Schrittes des erfindungsgemäßen Verfahrens setzt man bezogen auf 1 Mol des im ersten Schritt eingesetzten 2,4,5-Trifluor-anilins der Formel (II) im allgemeinen zwischen 1 und 10 Mol, vorzugsweise zwischen 2 und 8 Mol eines Alkalimetallcyanids und zwischen 0,01 und 0,5 Mol, vorzugsweise zwischen 0,05 und 0,2 Mol einer Übergangsmetallverbindung ein.

In einer bevorzugten Ausführungsform des zweiten Schrittes des erfindungsgemäßen Verfahrens wird die gemäß dem ersten Schritt erhaltene Reaktionsmischung unter Rühren zu einer wässrigen Lösung von Alkalimetallcyanid und Übergangsmetallverbindung gegeben, wobei durch gleichzeitige Zugabe eines Säureakzeptors der pH-Wert annähernd im Neutralbereich gehalten wird. Die Reaktionsmischung wird dann weiter gerührt, bis die Umsetzung praktisch abgeschlossen ist.

Vorzugsweise wird das im ersten Schritt erhaltene Reaktionsprodukt vor der Umsetzung im zweiten Schritt nicht isoliert.

Die Aufarbeitung und die Isolierung des Reaktionsproduktes der Formel (I) kann auf übliche Weise erfolgen. Beispielsweise wird mit einem mit Wasser praktisch nicht mischbaren organischen Lösungsmittel, wie z.B. Essigsäureethylester, geschüttelt und von der organischen Extraktionslösung dann das Lösungsmittel unter vermindertem Druck sorgfältig abdestilliert, wobei das Produkt der Formel (I) als Rückstand verbleibt.

Die nach dem erfindungsgemäßen Verfahren herzustellende Verbindung 2,4,5-Trifluor-benzonitril der Formel (I) kann als Zwischenprodukt zur Herstellung von Wirkstoffen im Bereich der Medizin und der Landwirtschaft verwendet werden (siehe EP-A-191 185, EP-A-597 360, EP-A-617 026, EP-A-654 468).

### Herstellungsbeispiele:

### Beispiel 1

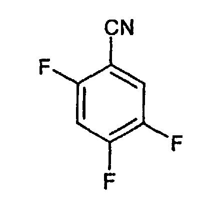

4,4 g (30 mMol) 2,4,5-Trifluor-anilin werden in 25 ml Essigsäure ("Eisessig") gelöst und mit einem Eisbad gekühlt. Dann werden unter Rühren 4,5 g (31,5 mMol) Nitrosylschwefelsäure dazu gegeben und die Reaktionsmischung wird nach Entfernen des Eisbads noch eine Stunde gerührt (erster Schritt).

Die so erhaltene Diazoniumsalzlösung wird dann unter Rühren zu einer auf 5°C abgekühlten Lösung von 7,8 g (160 mMol) Natriumcyanid und 0,3 g (3 mMol) Kupfer-(I)-cyanid in 40 ml Wasser gegeben. Durch gleichzeitige Zugabe von ca. 80 ml einer 25%igen wässrigen Lösung von Natriumcarbonat wird der pH-Wert angenähert im Neutralbereich gehalten. Anschließend wird die Reaktionsmischung noch ca. 15 Minuten gerührt und dann zweimal mit je 100 ml Essigsäureethylester extrahiert. Von den vereinigten organischen Extraktionslösungen wird das Lösungsmittel im Wasserstrahlvakuum sorgfältig abdestilliert.

Man erhält 4,1 g (92 %iges Produkt nach gaschromatografischer Analyse, d.h. 80 % der Theorie) 2,4,5-Trifluor-benzonitril als öligen Rückstand.

## Patentansprüche

1. Verfahren zum Herstellen von 2,3,5-Trifluor-benzonitril, **gekennzeichnet durch** die folgenden Schritte:
A) Umsetzen von 2,4,5-Trifluor-anilin mit einen Nitrosierungsmittel in Gegenwart eines Verdünnungsmittels;
B) Umsetzen des im Schritt A) erhaltenen Reaktionsproduktes mit einem Alkalimetallcyanid in Gegenwart einer Übergangsmetallverbindung, wobei die Übergangsmetallverbindung zwischen 0,01 und 0,5 Mol bezogen auf 1 Mol des im ersten Schritt eingesetzten 2,4,5-Trifluor-anilins eingesetzt wird, eines Säureakzeptors und eines Verdünnungsmittels.

2. Verfahren gemäß Anspruch 1, **dadurch gekennzeichnet, dass** man in Schritt B) das in Schritt A) erhaltene Reaktionsprodukt zu einer wässrigen Lösung von Alkalimetallcyanid und Übergangsmetallverbindung zugibt unter gleichzeitiger Zudosierung eines Säureakzeptors, so dass der pH-Wert der wässrigen Lösung im Neutralbereich gehalten wird.

3. Verfahren nach einem der Ansprüche 1 oder 2, **dadurch gekennzeichnet, dass** die Umsetzung im Schritt B) in Gegenwart einer Übergangsmetallverbindung zwischen 0,05 und 0,2 Mol bezogen auf 1 Mol des im Schritt A) eingesetzten 2,4,5-Trifluor-anilins durchgeführt wird.

4. Verfahren nach einem der Ansprüche 1 bis 3, **dadurch gekennzeichnet, dass** die Umsetzung im Schritt A) bei einer Temperatur zwischen -20°C und +30°C, vorzugsweise zwischen -10°C und +20°C, durchgeführt wird und die Umsetzung im Schritt B) bei einer Temperatur zwischen -10°C und +40°C, vorzugsweise zwischen 0°C und +30°C, durchgeführt wird.

5. Verfahren nach einem der Ansprüche 1 bis 4, **dadurch gekennzeichnet, dass** als Nitrosierungsmittel Alkalimetallnitrite, Alkylnitrite und/oder Nitrosylschwefelsäure eingesetzt wird.

6. Verfahren nach Anspruch 5, **dadurch gekennzeichnet, dass** als Nitrosierungsmittel Nitrosylschwefelsäure eingesetzt wird.

7. Verfahren nach einem der Ansprüche 1 bis 6, **dadurch gekennzeichnet, dass** als Alkalimetallcyanid Natriumcyanid und/oder Kaliumcyanid eingesetzt wird.

8. Verfahren nach einem der Ansprüche 1 bis 7, **dadurch gekennzeichnet, dass** als Übergangsmetallverbindung eine Kupferverbindung, vorzugsweise Kupfercyanid und/oder Kupfersulfat eingesetzt wird.

9. Verfahren nach einem der Ansprüche 1 bis 8, **dadurch gekennzeichnet, dass** als Säureakzeptor Alkalimetall- und/oder Erdalkalimetallcarbonate und/oder - hydrogencarbonate, vorzugsweise Natrium- und/oder Kalium(hydrogen)carbonat, eingesetzt wird.

10. Verfahren nach einem der Ansprüche 1 bis 9, **dadurch gekennzeichnet, dass** als Verdünnungsmittel ein oder mehrere inerte organische Lösungsmittel eingesetzt werden.

11. Verfahren nach Anspruch 10, **dadurch gekennzeichnet, dass** als Verdünnungsmittel im Schritt A) Carbonsäuren und im Schritt B) zusätzlich Wasser eingesetzt werden.

12. Verfahren nach einem der Ansprüche 1 bis 11, **dadurch gekennzeichnet, dass** das im Schritt A) erhaltene Reaktionsprodukt vor der Umsetzung im Schritt B) nicht isoliert wird.

## Claims

1. Process for preparing 2,3,5-trifluoro-benzonitrile, **characterized by** the following steps:
A) reaction of 2,4,5-trifluoro-aniline with a nitrosating agent in the presence of a diluent;
B) reaction of the reaction product obtained in step A) with an alkali metal cyanide in the presence of a transition metal compound which is employed in an amount between 0.01 and 0.5 mol per mole of the 2,4,5-trifluoro-aniline employed in the first step, an acid acceptor and a diluent.

2. Process according to Claim 1, **characterized in that**, in step B), the reaction product obtained in step A) is added to an aqueous solution of alkali metal cyanide and transition metal compound with simultaneous metered addition of an acid acceptor such that the pH of the aqueous solution is maintained in the neutral range.

3. Process according to Claim 1 or 2, **characterized in that** the reaction in step B) is carried out in the presence of between 0.05 and 0.2 mol of a transition metal compound per mole of the 2,4,5-trifluoro-aniline employed in step A).

4. Process according to any of Claims 1 to 3, **characterized in that** the reaction in step A) is carried out at a temperature between -20°C and +30°C, preferably between -10°C and +20°C, and the reaction in step B) is carried out at a temperature between -10°C and +40°C, preferably between 0°C and +30°C.

5. Process according to any of Claims 1 to 4, **characterized in that** the nitrosating agents used are alkali metal nitrites, alkyl nitrites and/or nitrosylsulphuric acid.

6. Process according to Claim 5, **characterized in that** the nitrosating agent used is nitrosylsulphuric acid.

7. Process according to any of Claims 1 to 6, **characterized in that** the alkali metal cyanide used is sodium cyanide and/or potassium cyanide.

8. Process according to any of Claims 1 to 7, **characterized in that** the transition metal compound used is a copper compound, preferably copper cyanide and/or copper sulphate.

9. Process according to any of Claims 1 to 8, **characterized in that** the acid acceptors used are alkali metal carbonates and/or alkaline earth metal carbonates and/or alkali metal bicarbonates and/or alkaline earth metal bicarbonates, preferably sodium (bi)carbonate and/or potassium (bi)carbonate.

10. Process according to any of Claims 1 to 9, **characterized in that** the diluent used comprises one or more inert organic solvents.

11. Process according to Claim 10, **characterized in that** the diluents used in step A) are carboxylic acids and in step B) are additionally water.

12. Process according to any of Claims 1 to 11, **characterized in that** the reaction product obtained in step A) is not isolated prior to the reaction in step B).

## Revendications

1. Procédé de production du 2,3,5-trifluoro-benzonitrile, **caractérisé par** les étapes suivantes :
A) réaction de la 2,4,5-trifluoraniline avec un agent de nitrosation en présence d'un diluant ;
B) réaction du produit réactionnel obtenu dans l'étape A) avec un cyanure de métal alcalin en présence d'un composé de métal de transition utilisé en proportion comprise entre 0,01 et 0,5 mole pour 1 mole de la 2,4,5-trifluoraniline utilisée dans la première étape, d'un accepteur d'acide et d'un diluant.

2. Procédé suivant la revendication 1, **caractérisé en ce qu'**on ajoute dans l'étape B) le -produit réactionnel obtenu dans l'étape A) à une solution aqueuse de cyanure de métal alcalin et de composé de métal de transition en même temps qu'on ajoute un accepteur d'acide, de manière que la valeur de pH de la solution aqueuse soit maintenue dans la région neutre.

3. Procédé suivant l'une des revendications 1 et 2, **caractérisé en ce que** la réaction dans l'étape B) est conduite en présence d'un composé de métal de transition en proportion comprise entre 0,05 et 0,2 mole pour 1 mole de la 2,4,5-trifluoraniline utilisée dans l'étape A).

4. Procédé suivant l'une des revendications 1 à 3, **caractérisé en ce qu'**on conduit la réaction dans l'étape A) à une température comprise entre -20°C et +30°C, de préférence enbre -10°C et +20°C et qu'on conduit la réaction dans l'étape B) à une température comprise entre -10°C et +40°C, de préférence entre 0°C et +30°C.

5. Procédé suivant l'une des revendications 1 à 4, **caractérisé en ce qu'**on utilise comme agent de nitrosation des nitrites de métaux alcalins, des nitrites d'alkyle et/ou de l'acide nitrosylsulfurique.

6. Procédé suivant la revendication 5, **caractérisé en ce qu'**on utilise comme agent de nitrosation l'acide nitrosylsulfurique.

7. Procédé suivant l'une des revendications 1 à 6, **caractérisé en ce qu'**on utilise comme cyanure de métal alcalin du cyanure de sodium et/ou du cyanure de potassium.

8. Procédé suivant l'une des revendications 1 à 7, **caractérisé en ce qu'**on utilise comme composé de métal de transition un composé de cuivre, de préférence du cyanure de cuivre et/ou du sulfate de cuivre.

9. Procédé suivant l'une des revendications 1 à 8, **caractérisé en ce qu'**on utilise comme accepteur d'acide des carbonates et/ou des bicarbonates de métaux alcalins et/ou de métaux alcalino-terreux, de préférence du carbonate et/ou du bicarbonate de sodium et/ou de potassium.

10. Procédé suivant l'une des revendications 1 à 9, **caractérisé en ce qu'**on utilise comme diluant un ou plusieurs solvants organiques inertes.

11. Procédé suivant la revendication 10, **caractérisé en ce qu'**on utilise comme diluant des acides carboxyliques dans l'étape A) et en outre de l'eau dans l'étape B).

12. Procédé suivant l'une des revendications 1 à 11, **caractérisé en ce que** le produit réactionnel obtenu dans l'étape A) n'est pas isolé avant la réaction dans l'étape B).
